# EUROPEAN PATENT SPECIFICATION

(11) **EP 2 611 440 B1**
(45) Date of publication and mention of the grant of the patent: **01.02.2017**
(21) Application number: 11832859.0
(22) Date of filing: 31.08.2011
(51) Int. Cl.: A61K 31/27, A61K 31/16, A61K 31/535, A61K 31/275, A61P 25/36, A61P 25/00

(54) **TREATMENT FOR COCAINE ADDICTION**
BEHANDLUNG VON KOKAINSUCHT
TRAITEMENT CONTRE LA COCAÏNOMANIE

(30) Priority: 01.09.2010 US 379095 P
(43) Date of publication of application: 10.07.2013
(60) Divisional application: 16204193.3
(73) Proprietor: Tonix Pharmaceuticals, Inc., New York, NY 10022 (US)
(72) Inventor: LEDERMAN, Seth, New York, NY 10022 (US); HARRIS, Herbert, New York, NY 10022 (US)
(74) Representative: Lau, Sarah Jane
(86) International application number: PCT/US2011/001529
(87) International publication number: WO 2012/050594

(56) References cited:
- WO-A2-03/037313
- US-A1- 2003 087 814
- US-A1- 2006 018 933
- US-A1- 2008 078 382
- ELKASHEF ET AL: "Double Blind, placebo controlled trial of Selegiline transdermal system for the treatment of cocaine dependency", DRUG AND ALCOHOL DEPENDENCE, vol. 85, 2006, - 2006, pages 191-197, XP002718314,
- KOSTEN ET AL: "Ther Potential of dapamine agonists in drug addiction.", EXPERT OPIN. INVEST. DRUGS, vol. 11, no. 4, 2002, - 2002, pages 491-499, XP002718315,
- WIESBECK ETAL: "Neue Entwicklungen in der Pharmakotherapie der Kokainabhängigkeit.", DER NERVENARZT, vol. 9, 2006, - 2006, pages 1064-1070, XP002718316,

## Description

### BACKGROUND

All references cited in this specification, and their references, are incorporated by reference herein in their entirety where appropriate for teachings of additional or alternative details, features, and/or technical background.

### CROSS-REFERENCE TO RELATED APPLICATIONS:

The present application which claims priority from U.S. Provisional Patent Application No. 61/379,095, filed September 1, 2010, is a continuation in part from U.S. Patent Application Serial No. 12/145,792, filed June 25, 2008, which is a divisional of U.S. Patent Application Serial No. 10/287,153, filed November 4, 2002 (abandoned), which claims the benefit of the filing date of U.S. Provisional Patent Application No. 60/338,901, filed November 5, 2001.

### BACKGROUND OF THE INVENTION

### 1. Field of the Invention:

The present invention relates to compositions and methods for preventing, ameliorating or treating addiction to cocaine, alcohol and similar nerve or psychostimulants. Such compositions and methods may be used to facilitate drug use cessation, and may comprise a combination of aldehyde dehydrogenase inhibitors and monoamine oxidase inhibitors; more particularly, the treatment may include a single dosage unit of such combined active ingredients. Such compositions will reduce pleasurable experiences associated with use of alcohol, cocaine, or stimulants. In addition, such compositions will produce unpleasant or aversive experiences when used with alcohol, cocaine, or stimulants. Lastly, such compositions have mildly reinforcing properties that may enhance compliance with their use in subjects prone to substance abuse.

### 2. Description of the Related Art:

Cocaine, stimulants, and alcohol are recognized as the most commonly abused drugs. According to the Diagnostic and Statistical Manual of Mental Disorders (DSM-IV), problematic alcohol use is divided into alcohol abuse and alcohol dependence. Cocaine abuse and dependence remains a substantial problem in the United States of America. Stimulants are drugs that tend to increase alertness and physical activity. The groups include pharmaceuticals such as amphetamines and the street drugs commonly called "uppers" or "speed," and cocaine. Specific examples include cocaine, methamphetamine, amphetamines, methylphenidate, nicotine, and MDMA (3,4-methylenedioxymethamphetamine), better known as "Ecstasy." Clinically significant abuse or dependence on these substances is classified by the Diagnostic and Statistical Manual (DSM-IV) as follows: Amphetamine abuse (305.70)/ Amphetamine dependence (304.40); Cocaine abuse (305.60)/Cocaine dependence (304.20); Phencyclidine abuse (305.90)/dependence (304.90); Nicotine dependence (305.1) (Amperican Psychiatric Association, 2000).

Cocaine dependence has developed into a public health problem with negative medical, social, and economic effects. For example, in recent years cocainerelated emergency room visits increased almost 50%. Cocaine addiction or dependence affected approximately 2.4 million people in the United States in 2005. In the sense of this invention the term "addiction" may be defined as a compulsive drug taking or abuse condition related to "reward" system of the afflicted patient. The treatment of cocaine addiction or dependency has targeted a lowering of dopaminergic tone to help decrease or attenuate the "reward effect. Behavioral interventions may help in treating cocaine addiction, but have not yet resulted in approved medications to treat these disorders despite many years of study.

Moreover, cocaine users tend to imbibe alcohol concurrently to mellow the psychological anxiety and hyperagitation frequently associated with chronic use of cocaine. It appears that almost 90% of cocaine abusers are also dependent on alcohol. The consumption of both cocaine and alcohol has been suggested as reinforcing the dependency and toxicity in the formation of the metabolite, cocaethylene. Alcohol abuse and dependence commonly lead to other problems such as alcohol-related violence, motor vehicle accidents, and medical consequences of chronic alcohol ingestion including death.

The enzyme aldehyde dehydrogenase (ADLH) inhibitor disulfiram which is also dopamine-beta-hydrolase (DBH) inhibitor has been reported effective in studies for reducing cocaine use (Carroll et al. Arch.Gen. Psychiatry 2004;29:1123-1128), although perhaps not suitable for all populations (Nich et al. Addict. Behavior 2004; 29:1123-1128). Selegiline, a monoamine oxidase (MAO)-B inhibitor, is known to block dopamine breakdown, thus increasing synaptic dopamine levels and inhibit dopamine re-uptake (Ebadi et al. J. Neurosci. Res. 2002; 67:285-289).

Although disulfiram and selegiline have been available on the market for many years, their combined use has never been studied systematically. Despite widespread use, a single case report has appeared in the literature involving a significant adverse event (transient delirium) associated with administration of an MAO inhibitor tranylcypromine in a patient with a disulfiram implant on therapeutic lithium (Blansjaar, B. A. 1995 Am J Psychiatry 152:296.) It should be noted that tranylcypromine is an inhibitor of MAO-A and -B. Selegiline selectively inhibits only the enzyme monoamine oxidase B (MAO-B) at the low dose of 10 mg/day or less. Moreover, the subject in the case report also had a therapeutic level of Li⁺ (0.7 mM/L in serum), factors which may have contributed to the delirium.

One of the pharmacotherapies that have been suggested for treating alcoholism, including facilitating alcohol cessation, is the administration of agents that inhibiting the enzyme aldehyde dehydrogenase (ALDH), an enzyme involved in the removal of acetaldehyde, a toxic metabolite of alcohol. Examples of ALDH inhibitors include, e.g., disulfiram, coprine, cyanamide, 1-aminocyclopropanol (ACP), daidzin, cephalosporins, antidiabetic sulfonyl ureas, metronidazole, and any of their metabolites or analogs exhibiting ALDH-inhibiting activity including, e.g., S-methyl N,N-diethyldithiocarbamate, S-methyl N,N-diethyldithiocarbamate sulfoxide, and S-methyl N,N-diethylthiocarbamate sulfoxide. Patients who consume such inhibitors of ALDH experience mild to severe discomfort if they ingest alcohol. The efficacy of therapies using ALDH inhibitors depends on the patient's own motivation to self-administer the ALDH inhibitors, e.g., oral forms of the inhibitors, or to receive additional therapies, e.g., DEPO forms of disulfiram. In fact, patient compliance is a significant problem with these types of therapies. Disulfiram therapy is known to require close medical supervision for maintenance of abstinence/relapse prevention. Some of the reasons for this difficulty are described in the following paragraphs.

Although multiple forms of ALDH exist, ALDH-I (also known as ALDH-2) and ALDH-II (also known as ALDH-1) are the major enzymes responsible for the oxidation of acetaldehyde. ALDH-I has a higher affinity for acetaldehyde than ALDH-II, and is thought to be the primary enzyme involved in alcohol detoxification [Keung, W. M., et al., Proc. Natl. Acad. Sci. USA 95:2198-2203 (1998)]. The discovery that 50% of the Asian population carries a mutation in ALDH-I that inactivates the enzyme, together with the low occurrence of alcohol abuse in this population supports the contention that it is this isozyme of ALDH that is primarily responsible for alcohol detoxification. Recent studies also implicate ALDH-I in the metabolism of monoamine neurotransmitters such as serotonin (5-HT) and dopamine (DA) [Keung, W. M., et al., Proc. Natl. Acad. Sci. USA 95:2198-2203 (1998)].

Disulfiram, also known as tetraethylthioperoxydicarbonic diamide, bis-diethylthiocarbamoyl disulfide, tetraethylthiuram disulfide, Cronetal.TM., Abstenil.TM., Stopetyl.TM., Contrain.TM., Antadix.TM., Anietanol.TM., Exhoran.TM., ethyl thiurad, Antabuse.TM., Etabuse.TM., RO-sulfiram, Abstinyl.TM., Thiuranide.TM., Esperal.TM., Tetradine.TM., Noxal.TM., Tetraeti.TM. [Swift, supra], is a potent irreversible inhibitor of ALDH-II and inhibits ALDH-I only slightly. Recent studies suggest that the inhibition of ALDH-I by disulfiram occurs indirectly via its metabolites, e.g., S-methyl-N,N-diethylthiocarbamate sulfoxide (DETC-MeSO) [Yourick et al., Alcohol 4:463 (1987); Yourick et al., Biochem. Pharmacol. 38:413 (1989); Hart et al., Alcohol 7:165 (1990); Madan et al., Drug Metab. Dispos. 23:1153-1162 (1995)]. Ingestion of alcohol while taking disulfiram results in the accumulation of aldehydes, which causes tachycardia, flushing, diaphoresis, dyspnea, nausea and vomiting (also known collectively as the disulfiram or disulfiram-ethanol reaction).

Although disulfiram has been available in the United States for many decades, patients frequently have difficulty complying with disulfiram treatment therapies. One reason for poor compliance is the lack of motivation for the patient to continue to take disulfiram, that is, other than self-motivation (i.e., there is no positive reinforcement for taking disulfiram). Another reason is because of the discomfort that arises if the patient ingests alcohol during disulfiram therapy [McRae et al., supra; Swift, R. M., supra; Kick, S., supra]. In fact, disulfiram has not proven to be useful in maintaining long-term sobriety [Kick, supra]. More recently, disulfiram has found use in the treatment of cocaine addiction. It has been shown to reduce cocaine abuse and relapse in several outpatient clinical trials ((Carroll, Fenton et al. 2004); (Carroll, Nich et al. 1998); (George, Chawarski et al. 2000); (Petrakis, Carroll et al. 2000)). Disulfiram is approved by the Food and Drug Administration for the treatment of alcohol dependence. The efficacy of this drug in alcohol dependency is due to aversive symptoms experienced when acetaldehyde levels increase following ethanol ingestion. Disulfiram also inhibits dopamine β-hydroxylase (DBH), the enzyme that converts dopamine to norepinephrine, and thereby causes synaptic dopamine levels to increase relative to norepinephrine (Karamanakos, Pappas et al. 2001)). Previous research involving disulfiram and cocaine found that disulfiram increased the rating of "nervousness" and "paranoia" from intranasal cocaine (Hameedi, Rosen et al. 1995) (McCance-Katz, Kosten et al. 1998). In a more recent study, (Baker, Jatlow et al. 2007) reported that disulfiram treatment, in addition to increasing aversive experiences associated with cocaine, also attenuated the "high" feelings, (Baker, Jatlow et al. 2007). Another possibility is that disulfiram, through inhibition of the enzyme DBH, may increase the amount of dopamine in the brain by blocking dopamine's conversion to norepinephrine and thereby increase the dopamine that amphetamine releases (Karamanakos, Pappas et al. 2001). As the concurrent abuse of cocaine with alcohol is both increasingly common, it has become an intractable clinical problem for pharmacotherapeutic approaches. The problem of treatment either alcohol or cocaine abuse or both is one of efficacy and compliance.

Coprine (N5-(hydroxycyclopropyl)-L-glutamine) has been shown to inhibit ALDH via its active metabolite, 1-aminocyclopropanol (ACP). U.S. Pat. No. 4,076,840 describes the synthesis and use of cyclopropyl benzamides, including coprine, for the treatment of alcoholism. In rat studies, coprine effectively suppressed ethanol consumption, and was shown to be a more potent inhibitor of ALDH as compared to disulfiram [Sinclair et al., Adv. Exp. Med. Biol. 132:481-487 (1980); U.S. Pat. No. 4,076,840].

Cyanamide has been described as an alcohol-sensitizing agent that is less toxic than disulfiram [Ferguson, Canad. M.A.J. 74:793-795 (1956); Reilly, Lancet 911-912 (1976)]. Although cyanamide is unable to inhibit either ALDH-I or ALDH-II *in vitro,* a reactive product of cyanamide catabolism inhibits both isozymes in vivo, indicating that cyanamide inhibits ALDH via a reactive species [DeMaster et al., Biochem. Biophys. Res. Com. 107:1333-1339 (1982)]. Cyanamide has been used for treating alcoholism but has not been approved in the U.S. Citrated calcium cyanamide is marketed in other countries as Temposil.TM., Dipsane.TM. and Abstem.TM., and plain cyanamide is marketed as Colme.TM. in Spain [See, U.S. Pat. No. 6,255,497].

Daidzin is a selective potent reversible inhibitor of ALDH-I, originally purified from an ancient Chinese herbal treatment for alcohol abuse. Its analogs include daidzein-7-O-[.omega.-carboxynonyl] ether (deczein), daidzein-7-O-[.omega.-carboxyhexyl] ether (hepzein), daidzein-7-O-[.omega.-carboxypentyl ether (hexzein), daidzein, puerarin, and dicarboxymethyl-daidzein [Keung, Chemico-Bio. Int.130-132:919-930 (2001)]. U.S. Pat. Nos. 5,204,369; 5,886,028; 6,121,010; and 6,255,497 describe methods for treating alcohol dependence or abuse using these compounds.

One of the major problems associated with therapies using ALDH inhibitors is ensuring patient compliance with the regimen. According to applicant's knowledge, there have been no teachings that suggest pharmacotherapies that adequately address this problem. For example, WO 99/21540 describes the administration of disulfiram in combination with compounds that bind to the D1 and/or D5 receptors and mimic dopamine to reduce craving for addictive substances in mammals. However, WO 99/21540 does not suggest pharmacotherapy for ensuring patient compliance with the regimen, which is important for the success of the treatment.

Another pharmacotherapy that has been suggested for treating alcoholism or cocaine addiction involves the inhibition of monoamine oxidases (MAOs). MAOs catalyze the oxidation of a variety of monoamines, including epinephrine, norepinephrine, serotonin and dopamine. MAOs are iron containing enzymes that exist as two isozymes A (MAOA) and B (MAOB). Various publications have described treatments for alcoholism using MAOB inhibitors [e.g, WO 92/21333, WO 96/37199]. WO 96/35425 discusses a treatment for alcoholism using a selective MAOB inhibitor in combination with a partial agonist of the 5-TH1A receptor. WO 00/71109 discusses a treatment for alcohol withdrawal symptoms using the MAOB inhibitor desmethylselegiline in combination with a second drug that treats alcohol withdrawal symptoms. U.S. Pat. No. 6,239,181 describes methods for alleviating symptoms associated with alcoholic neuropathy by administering the MAOB inhibitor, selegiline. However, none of the above references teach or suggest the use of MAOB inhibitors in therapies using ALDH inhibitors. Moreover, none of these references teach that MAOB inhibitors have a sustained effect on ensuring patient compliance with other therapies.

Although cocaine addiction has been treated with either a MOAB inhibitor or a DBH inhibitor, no treatment has been reported involving a combination of these distinct pharmacological agents. Therefore, the invention provides a novel combination disulfiram/selegiline capsule treatment for cocaine abuse. Disulfiram and selegiline have each shown single-agent activity in preclinical and clinical studies of cocaine addiction and each act through potentially complimentary mechanisms. Advantages of the combination disulfiram/selegiline (over disulfiram alone or selegiline alone) may result from mechanistic synergy. Advantages of the combination over the single agents may result from improved compliance with prescribed regimen, particularly, given the particular challenges of adherence in treating substance abuse disorders. For example, the selegiline component of a disulfiram/selegiline combination drug may foster adherence to disulfiram, which may be effective when patients are compliant.

It is therefore an object of the present invention to provide a suitable combination composition and method for treating or reducing addiction to cocaine.

### SUMMARY

Aspects of the invention disclosed herein include a stimulant effect controlling composition comprising a combination of a monoamine oxidase (MOAB) inhibiting component and an aldehyde dehydrodrogenase (ALDH) inhibiting component.

Aspects of the invention disclosed herein include a composition comprising a cocaine therapeutic combination of a monoamine oxidase (MOAB) inhibiting component and an aldehyde dehydrodrogenase (ALDH) inhibiting component.

An aspect of the invention disclosed herein include a composition comprising a cocaine therapeutic combination of selegiline as a monoamine oxidase (MOAB) inhibiting component and an aldehyde dehydrodrogenase (ALDH) inhibiting component.

Another aspect of the invention disclosed herein include a composition comprising a cocaine therapeutic combination of selegiline as a monoamine oxidase (MOAB) inhibiting component and an aldehyde dehydrodrogenase (ALDH) inhibiting component, wherein the composition is formulated in unit dosage form.

Aspects of the invention disclosed herein include a compliance enhancing cocaine therapeutic composition comprising a combination of selegiline as a monoamine oxidase (MOAB) inhibiting component and an aldehyde dehydrodrogenase (ALDH) inhibiting component, wherein the composition is formulated in unit dosage form.

A further aspect of the invention include a single dosage unit composition including an activity wherein the aldehyde dehydrogenase inhibitor inhibits or reduces the activity of aldehyde dehydrogenase-1.

Another aspect of the invention provides the aldehyde dehydrogenase inhibitor selected from disulfiram, coprine, cyanamide, 1-aminocyclopropanol, daidzin, cephalosporin, antidiabetic sulfonyl urea, metronidazole, and metabolites or analogs thereof that exhibit aldehyde dehydrogenase-inhibiting activity.

A further aspect of the invention provides a cocaine therapeutic composition wherein the aldehyde dehydrogenase is disulfiram or a metabolite or analog thereof that exhibits aldehyde dehydrogenase-inhibiting activity.

A further aspect of the invention provides a composition wherein the composition comprises an amount of disulfiram selected from the group consisting of: about 500 mg, about 250 mg, about 125 mg and about 60 mg of disulfiram.

A further aspect of the invention provides a composition, which includes an amount of selegiline selected from the group consisting of: about 20 mg or less, about 15mg or less, about 10mg or less, about 5mg or less, about 2.5mg or less, and about 1mg or less of selegiline.

Another aspect of the invention provides a composition including the monoamine oxidase inhibitor B, selegiline, and the aldehyde dehydrogenase inhibitor, disulfiram.

Moreover, the invention provides a composition for treating alcohol dependency by administering a therapeutically effective amount of selegiline and about 60 mg to about 250 mg disulfiram; or a therapeutically effective amount of selegiline comprises about 5 mg to about 10 mg.

An aspect of the invention includes treatment of cocaine addiction by administering a composition comprising a combination of a monoamine oxidase (MOAB) inhibiting component and an aldehyde dehydrodrogenase (ALDH) inhibiting component.

An aspect of the invention includes treatment of cocaine addiction by administering a composition comprising a combination of a monoamine oxidase (MOAB) inhibiting component and an aldehyde dehydrodrogenase (ALDH) inhibiting component, wherein the composition is formulated in unit dosage form.

Another aspect of the invention includes treatment of cocaine addiction by administering a composition comprising a combination of selegiline as a monoamine oxidase (MOAB) inhibiting component and an aldehyde dehydrodrogenase (ALDH) inhibiting component selected from disulfiram, coprine, cyanamide, 1-aminocyclopropanol, daidzin, cephalosporin, antidiabetic sulfonyl urea, metronidazole, and metabolites or analogs thereof that exhibit aldehyde dehydrogenase-inhibiting activity.

Further aspects disclosed herein include a method for preventing, treating or reducing cocaine addiction in a patient in need for treatment thereof comprising the step of administering a therapeutically effective amount of a single dose of a composition comprising selegiline, a monoamine oxidase B inhibitor component, and an aldehyde dehydrogenase inhibitor component; in particular method wherein the aldehyde dehydrogenase inhibitor inhibits aldehyde dehydrogenase-I.

In addition, aspects of the invention include a method for preventing, treating or reducing cocaine addiction in a patient in need for treatment thereof, wherein the aldehyde dehydrogenase inhibitor includes disulfiram, coprine, cyanamide, 1-aminocyclopropanol, daidzin, cephalosporin, antidiabetic sulfonyl urea, metronidazole, or metabolites or analogs thereof exhibiting aldehyde dehydrogenase-inhibiting activity.

A further aspect of the invention provides a method for preventing, treating or reducing cocaine addiction in a patient in need for treatment thereof, wherein the aldehyde dehydrogenase inhibitor is disulfiram, or a metabolite or analog thereof that exhibits aldehyde-dehydrogenase-inhibiting activity.

A further aspect of the invention provides a method for preventing, treating or reducing cocaine addiction in a patient in need for treatment thereof, wherein the amount of disulfiram administered to said patient per day is selected from the group consisting of about 500 mg, about 250 mg, about 125 mg and about 60 mg.

A further aspect of the invention provides a method for preventing, treating or reducing both cocaine addiction and alcohol abuse in a patient in need for treatment thereof, wherein the monoamine oxidase B inhibitor is selegiline.

Another aspect of the method for preventing, treating or reducing both cocaine addiction and alcohol abuse in a patient in need for treatment thereof wherein the amount of selegiline administered to said patient per day comprise 0.03 µg - 15 mg, 0.05 µg - 10 mg, 0.15 µg - 5mg, 1.0 µg -2.5 mg, 10 µg - 2.5 mg, 0.10 mg - 2.5 mg of selegiline.

A further aspect of the invention provides a method of treatment, wherein the monoamine oxidase inhibitor B is selegiline and the aldehyde dehydrogenase inhibitor is disulfiram.

Furthermore, an aspect of the invention provides for a method, wherein the composition is administered orally, parentally or transdermally; which treatment may be accomplished in the form of as a capsule, a tablet or a transdermal patch.

An aspect of the invention provides treatment of cocaine addiction, wherein the patient is a human.

Another aspect of the invention provides a method for increasing compliance of a patient with a therapeutic regimen including self-administration of therapeutically effective amount of an aldehyde dehydrogenase inhibitor formulated in a single dosage with a therapeutically effective amount of selegiline.

A further aspect of the therapeutic treatment with self administered single doasage of selegiline and disulfiram is directed to a patient who is considered cocaine dependent.

An important aspect of the treatment of cocaine addiction includes a human patient.

### DETAILED DESCRIPTION

The psychopharmacotherapy of cocaine dependence is a rapidly developing field of research that may soon produce efficacious medications. Psychopharmacopytherapy comprises a medication-based therapy using medication in order to treat the underlying pathophysiology of cocaine dependency. Expanding research on reward-related brain circuitry, which is acutely activated and chronically dysregulated by cocaine, has helped reveal the neurobiological features of cocaine dependence and is guiding pharmacologic strategies that have significant potential to improve clinical outcome. Cocaine dependence is a multifaceted disorder with distinct clinical components that may respond to different pharmacologic approaches. Pharmacologic strategies for this disorder include blocking euphoria, reducing withdrawal and negative mood symptoms, ameliorating craving, and enhancing the prefrontal cortical function that seems to be impaired in cocaine-dependent patients. One medication may not be sufficient to treat these diverse elements of cocaine dependence because preliminary studies report efficacy with medications that have opposite actions on reward-related circuits.

Dopaminergic agents, such as disulfiram, indirectly inhibit dopamine-beta-hydrolase which converts dopamine to norepinephrine. However, there may be a gender difference as it has been reported by Nich⁹⁶ that men experienced reduced cocaine use while women did not. Selegiline, a monoamine oxidase (MAO)-B enzyme inhibitor blocks the catabolic enzyme responsible for dopamine breakdown causing increased synaptic presence of dopamine, and further has amphetamine effects to enhance dopamine release and inhibiting its re-uptake.

Since disulfiram and selegiline are approved by the FDA for other indications (alcohol dependence and Parkinson's, respectively), combining the active ingredients from marketed products into a new pharmaceutical product and treatment gives this composition a higher probability of success than developing new chemical entities because safety issues are not expected. In addition, the fact that each single-agent has activity further reduces risk that the combination might lack efficacy.

Disulfiram has been shown to reduce cocaine abuse and relapse in several outpatient clinical trials (Carroll, K. 2004 Arch Gen Psychiatry 61:264; Carroll, K. M. 1998 Addiction 93:713; George, T. P. 2000 Biol Psychiatry 47:1080; Petrakis, I. L. 2000 Addiction 95:219.). In particular, disulfiram is approved by the FDA for the treatment of alcohol dependence, in which case it is an aversive agent with a well-understood mechanism of action. As an alcohol aversive agent, disulfiram inhibits acetaldehyde dehydrogenase, which results in increased acetaldehyde levels following ethanol ingestion. Increased acetaldehyde produces a range of clinical reactions from unpleasant symptoms to toxicity. Significantly less is known about the mechanism by which disulfiram decreases cocaine use. Previous research involving disulfiram and cocaine found that disulfiram increases the rating of "nervousness" and "paranoia" from intranasal cocaine (Hameedi, F. A. 1995 Biol Psychiatry 37:560; McCance-Katz, E. F. 1998 Biol Psychiatry 43:540.) In addition to increasing aversive experiences, disulfiram also attenuates the "high" feelings from cocaine (Baker, J. R. 2007 Drug Alcohol Depend 87:202.) These results show that disulfiram has effects on cocaine use that are independent of reducing co-morbid alcohol abuse, although reducing alcohol use can be beneficial in some cocaine-abusing patients. It is not known how disulfiram renders cocaine ingestion unpleasant or blunts cocaine "high", but these effects may relate to inhibition of dopamine β-hydroxylase (DBH), the enzyme that converts dopamine to norepinephrine, by which disulfiram may increase synaptic dopamine relative to norepinephrine (Karamanakos, P. N. 2001 Pharmacol Toxicol 88:106.)

Selegiline as an oral mediation has shown significant effects in the treatment of cocaine abuse. Oral selegiline (Eldepryl® - the pure isomer of Deprenyl®) is approved by FDA for the treatment of Parkinson's disease because it increases dopamine levels and transmission. Oral selegiline is also being investigated for treating negative symptoms in schizophrenia. Selegiline is an irreversible inhibitor of monoamine oxidase type B (MAO-B) and also has other actions that may be related to metabolites amphetamine and methamphetamine (Yasar, S. 1996 J Neural Transm Suppl 48:61.) Selegiline is dosed 5 mg bid (with breakfast and with lunch.) A controlled release (CR) formulation was developed by Pharmavene and studied for cocaine effects, but was not approved by FDA and is not available. The CR version of selegiline was designed to reduce side-effects (such as sleep disturbance, presumably from metabolites amphetamine and methamphetamine) rather than MAO-B inhibition, since the irreversible inhibition of MAO-B is a prolonged effect. Data from animal studies taken together support selegiline's record as a safe agent without potential abuse over a thousand-fold dosage range from 0.001-1.0 mg/kg (Yasar & Bergman, 1994; Yasar,et al. 1994). Clinical studies involving the administration of selegiline to humans have been conducted using dosages ranging from 10mg to 60mg daily, for example: major depression (60 mg X 3 weeks) (Sunderland et al., 1994), atypical depression (20 mg X 6 weeks) (Quitkin et al., 1984), Alzheimer's disease (10 and 40 mg) (Tariot et al., 1987), nicotine dependence (10 mg X 4 weeks) (Houtsmuller, et al. 2002), and cocaine dependence (20 and 40 mg X 10 days) (Houtsmuller et al., 2004).

Several small studies have demonstrated effects of selegiline on subjective responses to cocaine administration. A study involving eight subjects showed that treatment with selegiline (10 mg CR formulation) was associated with decreased self-reported 'high' and 'stimulated' feelings after cocaine administration, measured as the area under the curve (Newton, T. F. 1999 Psychiatry Res 87:101.) A study employing PET scanning of subjects treated with selegiline 10 mg po/day followed by acute cocaine infusion showed that selegiline produced a 40% reduction in subjective euphoria which was accompanied by normalization of glucose utilization in limbic structures (Bartzokis, G. 1999 Neuropsychopharmacology 20:582.) In a unpublished double-blind, randomized study of 134 subjects with cocaine dependence (Bridge et al., 1999, unpublished NIH/NIDA Study Report - reviewed in Elkashef, A. 2006 Drug Alcohol Depend 85:191) selegiline 10 mg CR showed a statistically significant effect by *post hoc* analyses using a composite score of urine benzoylecgonine (BE), self report and observed improvement (p=0.12) (Elkashef, A. 2006, ibid).

Transdermal selegiline was developed to bypass GI MAO-A detoxification and hepatic first pass metabolism and is FDA approved for the treatment of depression (Emsam®). Transdermal selegiline was found to significantly blunt the "cocaine high" compared to placebo in an acute administration paradigm (Houtsmuller, E. J. 2004 Psychopharmacology (Berl) 172:31.) A subsequent study employing transdermal selegiline showed less consistent changes in subjective responses. (Harris, D. S. 2009 BMC Clin Pharmacol 9:13.) More recently, a larger multisite double-blind placebo-controlled study assessing the efficacy of transdermal selegiline was conducted on 300 cocaine dependent subjects and did not show a significant effect over placebo (Elkashef, A. 2006, ibid.) Of interest, the authors suggested that, among other explanations for the negative result, transdermal selegiline may be less effective than oral selegiline-CR because of differential production of selegiline metabolites including amphetamine and methamphetamine, which may play a role in substituting for cocaine during periods of abstinence (Elkashef, A., 2006 ibid; Yasar, S. 2005 Psychopharmacology (Berl) 182:95.)

In one embodiment, the MAOB inhibitor is selected from the group consisting of selegiline (Jumex.RTM., Jumexal.RTM. Carbex.RTM., Eldepryl.RTM., Movergan.RTM.; Aptapryl.RTM., AnipryI.RTM.; Eldeprine.RTM.; Plurimen.RTM.), desmethylselegiline, pargyline (Eudatin.RTM., Supirdyl.RTM;, Eutonyl.RTM.) [U.S. Pat. No. 3,155,584], rasagiline [R(+)N-propargyl-laminoindan], 3-N-phenylacetylamino-2,5-piper- idinedione, caroxyazone, AGN-1135 [WO 92/21333], MDL 72195 [WO 92/21333], J 508 [WO 92/21333], lazabemide [WO 00/45846], milacemide [WO 00/45846], IFO [WO 00/45846], mofegiline [WO 00/45846], and 5-(4-(4,4,4-trifluorobut- oxy)phenyl)-3-(2-methoxyethyl)-1,3,4-oxadiazol-2(3H)-one [WO 00/45846]. In another embodiment, prodrugs or metabolites of the MAOB inhibitors are contemplated. Said metabolite should have substantially the same or better selective MAOB inhibitor activity as its unmetabolized form.

A prodrug of a MAOB inhibitor is a derivatized MAOB inhibitor that is metabolized in vivo into the active inhibitory agent. Prodrugs according to this invention preferably have substantially the same or better therapeutic value than the underivatized MAOB inhibitor. For example, a prodrug useful according to this invention can improve the penetration of the drug across biological membranes leading to improved drug absorption; prolong duration of the action of the drug, e.g., slow release of the parent drug from the prodrug and/or decrease first-pass metabolism of the drug; target the drug action; improve aqueous solubility and stability of the drug (e.g., intravenous preparations, eyebrows etc.); improve topical drug delivery (e.g., dermal and ocular drug delivery); improve the chemical and/or enzymatic stability of drugs (e.g., peptides); or decrease side effects due to the drug. Methods for making prodrugs are readily known in the art.

The term "MAOB inhibitor" according to this invention or metabolite thereof, as used herein includes pharmaceutically acceptable salts of those compounds. Pharmaceutically acceptable salts of MAOB inhibitors useful according to the methods of this invention are salts prepared from pharmaceutically acceptable reagents. In one embodiment, said pharmaceutically acceptable salt is a hydrochloride salt.

Methods known in the art for evaluating the activity of MAOB and MAOA can be used for selecting MAOB inhibitors according to this invention. For example, blood samples can be drawn to determine platelet MAO activity using radiolabelled benzylamine or phenylethylamine. (i.e., evaluating MAOB inhibitory activity). [Murphy, D. L., et al., Psychopharm. 62:129-132 (1979); Murphy, D. L., et al., Biochem. Med. 16:254-265 (1976); all incorporated by reference herein] In one embodiment, MAOB activity is decreased greater than 80% compared to MAOB enzyme activity before treatment. In a preferred embodiment, MAOB activity is decreased greater than 90% or 95% compared to MAOB activity before treatment.

MAOA inhibitory activity can, for example, be evaluated by measuring levels of 3-methoxy-4-hydroxyphenylglycol (MHPG) or 5-hydroxyindoleacetic acid (5-HIAA) in the plasma of blood or in cerebral spinal fluid (CSF) by using gas chromatography-mass spectroscopy (gc-ms). [Murphy, D. L., et al., Clinical Pharmacology in Psychiatry, 3rd Series., Eds. Dahl, Gram, Paul, and Potter, Springer-Verlag: 1987; Major, L. F., et al., J. Neurochem. 39:229-231 (1979); Jimerson, D. C., et al., Biomed. Mass. Spectrom. 8:256-259 (1981); all incorporated by reference herein]. In one embodiment, after administration of the MAOB inhibitor, plasma MHPG levels should not be reduced lower than 45% of pretreatment levels of plasma MHPG. In a preferred embodiment, after administration of the MAOB inhibitor, plasma MHPG or CSF 5-HIAA levels should not be reduced more than 80% of pretreatment levels of MHPG or 5-HIAA levels, respectively.

ALDH inhibitors according to the invention are compounds that are capable of inhibiting the activity of one or more of the several isozymes of ALDH, e.g., ALDH-I and ALDH-II. According to one embodiment, the ALDH is involved in alcohol metabolism. ALDH inhibitors according to this invention include, e.g., disulfiram, coprine, cyanamide, 1-aminocyclopropanol (ACP), daidzin, cephalosporins, antidiabetic sulfonyl ureas, metronidazole, and any of their metabolites or analogs exhibiting ALDH-inhibiting activity. In another embodiment, the ALDH inhibitor is disulfiram or an ALDH-inhibiting metabolite thereof. Such metabolites include, e.g., S-methyl N,N-diethyldithiocarbamate, S-methyl N,N-diethyldithiocarbamate sulfoxide, and S-methyl N,N-diethylthiocarbamate sulfoxide.

The term "ALDH inhibitor" according to the invention or metabolite thereof, as used herein, includes pharmaceutically acceptable salts of those compounds.

The term cocaine addiction as substance abuse, according to the invention includes craving, drug seeking, self-administration, where the drug abuse is related to the form of the drug. ranging from coca leaves, coca paste, cocaine to crack. The term "alcoholism" according to the invention includes alcohol abuse and alcohol dependence as described below.

The term "alcohol abuse" is defined in the Diagnostic and Statistical Manual of Mental Disorders (DSM-IV). Alcohol abuse as a maladaptive pattern of alcohol use that leads to clinically significant impairment or distress. Symptoms include one or more of the following occurring within a 12-month period: (1) recurrent alcohol use that results in a failure to fulfill major role obligations at work, school or home; (2) recurrent alcohol use in physically hazardous situations; (3) recurrent alcohol-related legal problems; and (4) continued alcohol use despite having persistent or recurrent social or interpersonal problems caused or exacerbated by the effects of the substance [McRae et al., supra; Swift, R. M., supra; Kick, S., supra].

Alcohol dependence occurs when symptoms of abuse are accompanied by three or more of the following: (1) tolerance defined by either: (a) a need for markedly increased amounts of alcohol to achieve intoxication or desired effect, or (b) markedly diminished effect with continued use of the same amount of alcohol; (2) withdrawal manifested by either: (a) characteristic withdrawal syndrome for alcohol or (b) alcohol taken to relieve or avoid withdrawal symptoms; (3) alcohol taken in larger amounts over a longer period than as intended; (4) a persistent desire or unsuccessful efforts to reduce or control drinking; (5) much time spent in activities necessary to obtain alcohol, use alcohol, or recover from its effects; (6) important social, occupational, or recreational activities being given up or reduced because of drinking; and (7) continued use despite knowledge of having a persistent or recurrent physical or psychological problem caused or exacerbated by alcohol [McRae et al., supra; Swift, R. M., supra; Kick, S., supra].

Alcohol abuse or dependence can also result in other symptoms including dyspepsia or epigastric pain, headache, diarrhea, difficulty in sleeping, fatigue, unexplained weight loss, apparent malnutrition, easy bruising, increased mean corpuscular volume, elevated transaminase levels (especially an aspartate transaminase level greater than of alanine transaminase), elevated y-glutamyl transferase levels, iron-deficiency anemia, hepatomegaly, jaundice, spider angiomata, ascites, and peripheral edema. Behavioral symptoms associated with alcohol abuse or dependence include absenteeism from work or school, increasing irritability, difficulties with relationships, verbal or physical abuse, and depression [McRae et al., supra; Swift, R. M., supra; Kick, S., supra].

A patient to be treated for, or protected against, the onset of addiction to cocaine or alcohol or both according to this invention can be a human, including children and adults, who are susceptible to or are suffering from alcoholism or who are being treated for alcoholism and are susceptible to experiencing relapses. A patient who is having difficulty complying with, or is being induced to comply with, treatments using ALDH inhibitors or their active metabolites according to this invention can be a human, including children and adults.

The treating physician will know how to increase, decrease or interrupt treatment based upon the patient's response. Improvement for alcoholics or potentially relapsing alcoholics can be assessed by observing increased abstinence from consuming alcohol by the patient, following the methods of this invention, as compared to patients where therapy did not comprise the co-administration of a MAOB inhibitor. Improvement in compliance with self-administering ALDH inhibitors can be assessed by observing the increased duration over which patients, following the methods of this invention, take the ALDH inhibitor as compared to patients whose therapy did not comprise the co-administration of an MAOB inhibitor.

Any suitable route of administration can be employed for providing the patient with an effective dosage of a composition of this invention. For example, oral, peroral, buccal, nasal, pulmonary, vaginal, lingual, sublingual, rectal, parenteral, transdermal, intraocular, intravenous, intraarterial, intracardial intramuscular, intraperitoneal, intracutaneous, subcutaneous, sublingual, intranasal, intramuscular, and intrathecal administration and the like can be employed as appropriate. The term parenteral as used herein includes subcutaneous, intracutaneous, intravenous, intramuscular, intra-articular, intrasynovial, intrasternal, intrathecal, intralesional and intracranial injection or infusion techniques. According to one preferred aspect of this invention, the route of administration is the oral route.

The composition can be conveniently presented in unit dosage form and prepared by any of the methods well-known in the art of pharmacy. Dosage forms can include tablets, scored tablets, coated tablets, pills, caplets, capsules (e.g., hard gelatin capsules), troches, dragees, powders, aerosols, suppositories, parenterals, dispersions, suspensions, solutions, transdermal patches and the like, including sustained release formulations well known in the art. In one preferred embodiment, the dosage form is a scored tablet or a transdermal patch. U.S. Pat. No. 5,192,550, incorporated herein by reference, describes a dosage form for selegiline comprising an outer wall with one or more pores, in which the wall is impermeable to selegiline but permeable to external fluids. This dosage form can have applicability for oral, sublingual or buccal administration.

The compositions of this invention can be orally administered in any orally acceptable dosage form including, but not limited to, capsules, tablets, and aqueous suspensions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient (i.e., ALDH inhibitor and/or MAOB inhibitor) is combined with emulsifying and suspending agents. If desired, certain sweetening and/or flavoring and/or coloring agents can be added.

The compositions according to this invention can be in the form of a sterile injectable preparation, for example, as a sterile injectable aqueous or oleaginous suspension. This suspension can be formulated according to techniques known in the art using suitable dispersing or wetting agents (such as, for example, Tween 80) and suspending agents. The sterile injectable preparation can also be a sterile injectable solution or suspension in a non-toxic parenterally-acceptable diluent or solvent, for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium. For this purpose, any bland fixed oil can be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant such as Ph. Helv or a similar alcohol.

Methods for making transdermal patches including selegiline transdermal patches have been described in the art. [See e.g., U.S. Pat. Nos. 4,861,800; 4,868,218; 5,128,145; 5,190,763; and 5,242,950; and EP-A 404807, EP-A 509761, EP-A 593807, and EP-A 5509761,]

Compositions of this invention can also be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax and polyethylene glycols.

The compositions of this invention can be administered by nasal aerosol or inhalation. Such compositions are prepared according to techniques well-known in the art of pharmaceutical formulation and can be prepared as solutions in saline, employing benzyl alcohol or other suitable preservatives, absorption promoters to enhance bioavailability, fluorocarbons, and/or other solubilizing or dispersing agents known in the art.

Patients can be regularly evaluated by physicians, e.g., once a week, to determine whether there has been an improvement in symptoms and whether the dosage of the composition of the invention needs to be adjusted.

According to the methods of this invention, the MAOB inhibitor such as selegiline can be included in the composition comprising the ALDH inhibitor. Alternatively, the MAOB inhibitor can be administered simultaneously with the composition comprising the ALDH inhibitor, or at any time during the treatment of the patient with the ALDH inhibitor.

The various terms described above such as "therapeutically effective amount," are encompassed by the above-described dosage amounts and dose frequency schedule. Generally, a therapeutically effective amount of an MAOB inhibitor is that amount at which MAOB is inhibited but MAOA exhibits slight or no reduction in activity in the patient. Slight reduction in activity preferably comprises less than about 30% reduction in activity, more preferably less than about 20% reduction in activity, and yet more preferably less than about 10% reduction in activity. In one embodiment, the dosage of selegiline is an amount equal to or less than 15 mg per day. In another embodiment, the dosage of pargyline is equal to or less than 30 mg/day.

Throughout this specification, the word "comprise" or variations such as "comprises" or "comprising" will be understood to imply the inclusion of a stated integer or group of integers but not the exclusion of any other integer or group of integers.

The invention includes a novel treatment for cocaine abuse that combines disulfiram and selegiline, each of which has shown single-agent activity in decreasing cocaine use, particularly when selegiline is administered by mouth (po). Disulfiram and selegiline act through potentially complimentary mechanisms, which motivates the investigation into whether a fixed-dose, once-daily combination product may have additive or synergistic efficacy on acute and chronic cocaine abuse. Based on the activities of disulfiram and selegiline as single-agents, the combination product may provide a number of potentially beneficial effects for the treatment of cocaine addiction, including: 1) reduction of euphoric effects produced by cocaine; 2) enhancement of dysphoric effects produced by cocaine; 3) replacement of normal reward signals thought to be deficient in cocaine abusers and 4) mitigation of the negative symptoms that occur between cocaine binges.

Fixed-dose, once-daily combinations have the potential to enhance adherence (which is a challenge in treating addiction disorders) and to achieve effects with doses lower than those required of the individual components. Developing a fixed-dose combination requires pharmacokinetic study because disulfiram and selegiline may affect each other's metabolism and disulfiram (by inhibition of dopamine beta-hydroxylase (DBH)) may potentiate the activity of selegiline and of certain of selegiline's metabolites (i.e., amphetamine and methamphetamine) on increasing synaptic dopamine.

Combination of Disulfiram and Selegiline: Disulfiram and selegiline act through potentially synergistic mechanisms. The complexity of their mechanisms and of the metabolic pathways involved makes it difficult to predict specific neurobehavioral effects or drug-drug interactions. Selegiline increases noradrenergic and dopaminergic transmission; both by inhibiting MAO-B irreversibly and through the actions of its metabolites; amphetamine and methamphetamine. Whether or not the mechanism of the disulfiram single-agent activity on cocaine relates to DBH inhibition, disulfiram DBH inhibition is expected to modulate and possibly augment the increased dopaminergic effects of selegiline. The combination product may provide a number of potentially beneficial effects for the treatment of cocaine addiction, including: 1) reduction of euphoric effects produced by cocaine; 2) enhancement of dysphoric effects produced by cocaine; 3) replacement of normal reward signals thought to be deficient in cocaine abusers and 4) mitigation of the negative symptoms that occur between cocaine binges.

Treatment of cocaine addiction with combination of MOAB and DBH inhibitors: Patients addicted to cocaine are to receive formulations of cGMP disulfiram/selegine-CR capsules and evaluate pharmacokinetic and pharmacodynamic interactions (Year 1-2): A preferred capsule formulation for once-daily administration is intended to mimic the pharmacokinetic (PK) profiles of immediate release (IR) selegiline 5 bid (taken at breakfast and lunch) and disulfiram 250 mg qd. Once-daily dosing will be a clinically important feature optimizing adherence in cocaine abusing patients. In addition, the inventive treatment recapitulates the pharmacokinetic profile of selegiline-SR (by Pharmavene) that has shown clinical effects in cocaine abuse but is no longer available.

While selegiline is dosed bid while disulfiram is dosed once-daily, a controlled release formulation that can be dosed once-daily and will deliver consistent levels of each active ingredient that are bio-similar to the formulations which have shown effects on cocaine as single agents. Formulated disulfiram/selegiline; placebo/selegiline; disulfiram/placebo preparations may be tested by *in vitro* dissolution studies using simulated gastric and small-intestine fluid for human treatment. In addition, because there is a significant food effect (3-4 fold increase in absorption with food according to the Eldepryl® package insert) it is considered preferred to employ a formulation technology that forms micelles in the aqueous environment of the stomach, and creates consistent absorption either in the presence, or absence of food.

At the pharmacokinetic level, selegiline and disulfiram share common metabolic pathways that may alter plasma levels of one or both drugs. The metabolism of disulfiram is complex, involving at least four P-450 enzymes (CYP3A4, CYP1A2, CYP2A6, and CYP2D6) (Madan, A., et al. 1998 Alcohol Clin Exp Res 22(6):1212.) The metabolism of selegiline involves several CYP-450 enzymes including CYP2B6, CYP2C9, and CYP3A4/5. Both selegiline and its metabolite N-des methyl selegiline caused a concentration dependent inhibition of CYP2D6, CYP2C19 and CYP2B6 (Emsam® prescribing information).

A major objective of the present invention is to provide a treatment wherein any possible drug-drug interactions that may create safety concerns or confound efficacy may be avoided through an effective, yet nontoxic regimen that avoids patient delirium but enhances patient compliance, as discussed in the following examples.

### Example A:

Given the potential for drug-drug interactions, test combination formulations may be a reduced dose of 20% relative to doses in the FDA approved immediate release (IR) selegiline 5 bid and disulfiram 250 mg products.

### Example B:

Single unit dose medical compositions of disulfiram/selegiline-CR in cocaine users is selected for safety and tolerability and response to daily co-administration. Treatment protocol with a composition including the inventive combination of distinct active ingredients also provides optionally a test phase of a monotherapy phase during which the patient may be treated initially with either a dose of disulfiram or selegiline as seen appropriate by the physician. After about three to five days, treatment may be switched to the other active ingredient. After a total of seven to ten days of monotherapy, a patient is placed on a daily regimen with a single dose of disulfiram/selegiline-CR based on the efficacy of the monotherapies. As precaution, plasma levels of each drug are measured at regular daily, weekly and later monthly intervals monitor the patient throughout the course of the treatment. Platelet MAO-B activity and plasma DBH activity is similarly assayed throughout the treatment period as an indicator of effective enzyme inhibition. As part of the treatment, protocol provides that subjective and physiological responses are collected. In addition, physiological monitoring (heart rate, blood pressure) and subjective measurements (Profile of Mood States [POMS] and Visual Analogue Scale [VAS]) may be conducted before, during, and after each administration of the inventive composition. Optional measures of monoaminergic function will be obtained including prolactin, HVA, and urine HMPG.

### Example C:

The efficacy of the single unit dose disulfiram/selegiline-CR combination may be monitored over several weeks as necessary, evaluating the patients diagnosed with cocaine dependence. The results are best obtained by conducting an out-patient double-blind, randomized study on cocaine users under the inventive treatment that will measure: urine benzoylecgonine (BE); self reported cocaine use and adherence to therapy. In particular, cocaine use may be determined by evaluating the weekly mean proportion of cocaine non-use days confirmed by urine BE levels at each patient visit. Other measures such as the Addiction Severity Index and the Brief Substance Craving Scale will be secondary endpoints.

Patients can be regularly evaluated by physicians, e.g., once a week, to determine whether there has been an improvement in symptoms and whether the dosage of the composition of the invention needs to be adjusted.

According to the methods of this invention, the MAOB inhibitor can be included in the composition comprising the ALDH inhibitor. Alternatively, the MAOB inhibitor may be administered simultaneously with the composition comprising the ALDH inhibitor, or at any time as a single dosage during the treatment of the patient with the ALDH inhibitor.

The various terms described above such as "therapeutically effective amount," are encompassed by the above-described dosage amounts and dose frequency schedule. Generally, a therapeutically effective amount of an MAOB inhibitor is that amount at which MAOB is inhibited but MAOA exhibits slight or no reduction in activity in the patient. Slight reduction in activity preferably comprises less than about 30% reduction in activity, more preferably less than about 20% reduction in activity, and yet more preferably less than about 10% reduction in activity. In one embodiment, the dosage of selegiline is an amount equal to or less than 15 mg per day. In another embodiment, the dosage of selegiline is an amount equal to or less than 5 mg per day. In another embodiment, the dosage of pargyline is equal to or less than 30 mg/day.

### EXAMPLE 1

This investigation is to test the effective dose range for the selegiline moiety in combination with a disulfiram dosage held constant at 250mg a day. In a 14 day test series, the patient would take 250mg of disulfiram in combination with one of a series of increasing dosages of selegiline daily on awakening, e.g., starting with 1 mg selegeline. The only dietary precautions taken were avoidance of alcohol and unprocessed cheese. After 14 days, in the absence of untoward effects in the treated patient, the next selegiline dose in the series may be administered in combination with 250 mg of disulfiram for the next 14 days.

The above outlined protocol is followed with a treatment of a patient by applying the following selegiline dosages: 1mg, 5 mg, 10 mg, 20 mg, 30 mg, 40 mg, 50 mg and 60 mg. If no untoward side effects are noted with any of these combinations, the subject patient may remain treated on the disulfiram 250 mg / selegiline 60 mg for six months after which the dosage of the selegiline component of the combination can be tapered to 10mg for another six months and finally to a 5mg daily dose on an ongoing basis. If no undesirable side effects were encountered with any of these combinations, serial blood chemistry, complete blood counts, and liver function tests (all of which are taken before the treatment protocol is initiated) are taken after staying for 6 months on daily disulfiram 250 mg in combination with selegeline 60 mg, and again after 6 months on disulfiram 250 mg in combination with selegiline 5 mg. All tests remained at the expected levels.

### EXAMPLE 2

Based on such pretesting and other tests to determine the patient's condition a patient may undergo a regime of daily medication involving 250 mg disulfiram and 5 mg selegiline. Consequently, the efficacy of medication may be manifested by a significant change in attitude toward life and stimulant. Even after only one week on this regimen, the patient may find a much lower degree of anxiety and distraction which would normally interfere in his daily activities or tasks. Over a period several months the treatment on the combination of disulfiram and selegiline would show a distinctly greater efficacy than on disulfiram or selegeline alone.

With the therapeutically effective dosage, the treated patient's urge for stimulant would subside effectively. Thus, contrary to the usual return to drugs or stimulants, the treated patient is expected to sustain recovery and also maintain self-treatment requiring less frequent visits to the supervising physician or health professional. Since treatment dosage is also affected by the condition and weight/size of the individual person, a series of dose-ranging experiments may be conducted involving the very patient before the actual treatment regimen to discover whether, or at what level, selegiline would inhibit disulfiram-stimulant (cocaine) reaction if challenged with medically acceptable dose of cocaine. Alternatively, the regimen may be adjusted from standardized dosage-weight data or charts.

### EXAMPLE 3:

To determine an effective dosage, an experimental test series of 14 days of administering, e.g., 5 mg selegiline together with disulfiram in decreasing doses (i.e., 250mg, 125mg, 100mg, 75mg, 60mg, and 50mg) was followed by a single medically acceptable dose of cocaine. The lowest anti-stimulant effective disulfiram level was used in the combination with 5mg selegiline for the treatment dosage.

These protocol examples may demonstrate the pharmacological efficacy and safety of a treatment combining disulfiram with selegiline. In terms of efficacy, combined selegiline and disulfiram not only deters the cocaine dependent patient from resumption of the addiction associated activity but also lifts the patient's attitude into a more positive, significantly less compulsive frame of mind. The dose of selegiline at which these changes are observed is far below expected level, and may be specific to co-administration of aversive agents like disulfiram. Moreover, therapy of stimulant craving with an aversive agent has its own unique mental issues for patients who suffer from such addiction, and selegiline may seem to alleviate the ambivalence regarding discontinuing the chronic habit as well as having significant efficacy against the chronic anxiety so common in the state of early recovery from the drug. According to this invention a relatively low dosage of selegiline, in addition to the usual dosage of disulfiram, would improve patient's adherence and reaction to the treatment so as to reduce or eliminate drug dependence.

### EXPERIMENTAL EXAMPLE:

An investigation was undertaken to test the effective dose range for the selegiline moiety in combination with a disulfiram dosage held constant at 250mg a day. As a solo agent, selegiline is known to be safe and well tolerated, to have a generally high therapeutic index, and wide effective dose range. In a 14 day protocol, a subject patient took 250mg of disulfiram in combination with one of a series of increasing dosages of selegiline daily on awakening. The only dietary precautions taken were avoidance of alcohol and unprocessed cheese. After 14 days, if no untoward effects were experienced, the next selegiline dose in the series was administered in combination with 250mg of disulfiram for the next 14 days.

This protocol was followed with the following selegiline dosages: 10 mg, 20 mg, 30 mg, 40 mg, 50 mg and 60 mg. No untoward side effects were noted with any of these combinations. After completion of this study, the subject remained on the disulfiram 250 mg / selegiline 60 mg for six months after which the dosage of the selegiline component of the combination was tapered to 10mg for another six months and finally to a 5mg daily dose on an ongoing basis. No untoward side effects were noted with any of these combinations. Serial blood chemistry, complete blood counts, and liver function tests, all of which were taken before the Study protocol was initiated, and again after 6 months on disulfiram 250 mg in combination with selegeline 60 mg, and again after 6 months on disulfiram 250 mg in combination with selegiline 5 mg, all remained at the expected levels.

Throughout this specification, the word "comprise" or variations such as "comprises" or "comprising" will be understood to imply the inclusion of a stated integer or group of integers but not the exclusion of any other integer or group of integers.

### Statement Regarding Preferred Embodiments

While the invention has been described with respect to preferred embodiments, those skilled in the art will readily appreciate that various changes and/or modifications can be made to the invention without departing from the scope of the invention as defined by the appended claims. All documents cited herein are incorporated in their entirety herein.

## Claims

1. A stimulant effect controlling composition comprising a combination of selegiline as a monoamine oxidase (MOAB) inhibiting component and an aldehyde dehydrogenase (ALDH) inhibiting component, wherein the composition is formulated in unit dosage form; wherein the stimulant comprises cocaine.

2. The composition according to claim 1, wherein the aldehyde dehydrogenase inhibitor is an aldehyde dehydrogenase-I inhibitor selected from the group consisting of: disulfiram, coprine, cyanamide, 1-aminocyclopropanol, daidzin, cephalosporin, antidiabetic sulfonyl urea, metronidazole, and metabolites or analogs thereof that exhibit aldehyde dehydrogenase-I inhibiting activity.

3. The composition according to claim 1 or 2, wherein the aldehyde dehydrogenase is disulfiram or a metabolite or analog thereof that exhibits aldehyde dehydrogenase-I inhibiting activity.

4. The composition according to claim 1 or 3, wherein the composition comprises an amount of disulfiram selected from the group consisting of: about 500 mg, about 250 mg, about 125 mg, about 60 mg and about 30 mg of disulfiram.

5. The composition according to claim 1, wherein the composition comprises an amount of selegiline selected from the group consisting of: about 15 mg or less, about 10 mg or less, about 5 mg or less, about 2.5 mg or less, and about 1 mg or less of selegiline.

6. The composition according to claim 1, wherein the monoamine oxidase inhibitor B is selegiline and the aldehyde dehydrogenase-I inhibitor is disulfiram.

7. The composition according to claim 1, comprising a therapeutically effective amount daily of 5 mg to 10 mg selegiline and 60 mg to 250 mg disulfiram, respectively.

8. The composition according to claim 7, wherein the therapeutically effective daily amount of selegiline comprises 5 mg to 10 mg and the therapeutically effective daily amount of disulfiram comprises 250 mg.

9. A pharmaceutical composition comprising a therapeutically effective amount of a combination of 5 mg to 10mg selegiline as a monoamine oxidase (MOAB) inhibiting component and 60 mg to 250mg of disulfiram as an aldehyde dehydrogenase-I (ALDH) inhibiting component, wherein the composition is formulated in unit dosage form; and a pharmaceutically acceptable excipient or carrier.

10. A composition comprising 5 mg to 10 mg selegiline, a monoamine oxidase B inhibitor component, and 60 mg to 250 mg of disulfiram as an aldehyde dehydrogenase-I inhibitor component for use in a method for preventing, treating or reducing cocaine addiction in a patient in need.

11. The composition for use according to claim 10 , wherein the composition is administered orally, parentally or transdermally.

12. The composition for use according to claim 10 , wherein the composition is administered as a capsule, a tablet or a transdermal patch.

13. The composition for use according to claim 10, wherein the patient is a human.

14. Use of a composition comprising 5 mg to 10 mg selegiline, a monoamine oxidase B inhibitor component, and 60 mg to 250 mg of disulfiram as an aldehyde dehydrogenase-I inhibitor component in the manufacture of a medicament for preventing, treating or reducing cocaine addiction in a patient in need.

## Patentansprüche

1. Zusammensetzung zur Regulierung der Wirkung eines Stimulans, umfassend eine Kombination aus Selegilin als Monoaminoxidase (MOAB) inhibierende Komponente sowie eine Aldehyddehydrodrogenase (ALDH) inhibierende Komponente, wobei die Zusammensetzung in Einheitsdosierungsform formuliert ist; wobei das Stimulans Kokain umfasst.

2. Zusammensetzung nach Anspruch 1, wobei es sich bei dem Aldehyddehydrogenase-Inhibitor um einen Inhibitor von Aldehyddehydrogenase I handelt, der ausgewählt ist aus der Gruppe bestehend aus: Disulfiram, Coprin, Cyanamid, 1-Aminocyclopropanol, Daidzin, Cephalosporin, antidiabetischem Sulfonylharnstoff, Metronidazol und Metaboliten oder Analoga von diesen, die über eine Aldehyddehydrogenase I inhibierende Aktivität verfügen.

3. Zusammensetzung nach Anspruch 1 oder 2, wobei es sich bei der Aldehyddehydrogenase um Disulfiram oder einen Metaboliten oder ein Analogon von diesem handelt, der/das über eine Aldehyddehydrogenase I inhibierende Aktivität verfügt.

4. Zusammensetzung nach Anspruch 1 oder 3, wobei die Zusammensetzung eine Menge an Disulfiram umfasst, die ausgewählt ist aus der Gruppe bestehend aus: etwa 500 mg, etwa 250 mg, etwa 125 mg, etwa 60 mg und etwa 30 mg an Disulfiram.

5. Zusammensetzung nach Anspruch 1, wobei die Zusammensetzung eine Menge an Selegilin umfasst, die ausgewählt ist aus der Gruppe bestehend aus: etwa 15 mg oder weniger, etwa 10 mg oder weniger, etwa 5 mg oder weniger, etwa 2,5 mg oder weniger und etwa 1 mg oder weniger an Selegilin.

6. Zusammensetzung nach Anspruch 1, wobei es sich bei dem Inhibitor von Monoaminoxidase B um Selegilin und dem Inhibitor von Aldehyddehydrogenase I um Disulfiram handelt.

7. Zusammensetzung nach Anspruch 1, die jeweils eine therapeutisch wirksame tägliche Menge von 5 mg bis 10 mg an Selegilin und 60 mg bis 250 mg an Disulfiram umfasst.

8. Zusammensetzung nach Anspruch 7, wobei jeweils die therapeutisch wirksame tägliche Menge an Selegilin 5 mg bis 10 mg und die therapeutisch wirksame tägliche Menge an Disulfiram 250 mg umfasst.

9. Pharmazeutische Zusammensetzung, die eine therapeutisch wirksame Menge einer Kombination aus 5 mg bis 10 mg an Selegilin als Monoaminoxidase (MOAB) inhibierende Komponente sowie 60 mg bis 250 mg an Disulfiram als Aldehyddehydrodrogenase I (ALDH) inhibierende Komponente, wobei die Zusammensetzung in Einheitsdosierungsform formuliert ist; sowie einen pharmazeutisch verträglichen Exzipienten oder Träger umfasst.

10. Zusammensetzung, die 5 mg bis 10 mg an Selegilin als Monoaminoxidase B (MOAB) inhibierende Komponente und 60 mg bis 250 mg an Disulfiram als Aldehyddehydrodrogenase I inhibierende Komponente umfasst, zur Verwendung in einem Verfahren zur Prävention, Behandlung oder Reduktion von Kokainabhängigkeit bei einem Patienten, der dessen bedarf.

11. Zusammensetzung zur Verwendung nach Anspruch 10, wobei die Verabreichung der Zusammensetzung oral, parenteral oder transdermal erfolgt.

12. Zusammensetzung zur Verwendung nach Anspruch 10, wobei die Verabreichung der Zusammensetzung in Form einer Kapsel, einer Tablette oder eines transdermalen Pflasters erfolgt.

13. Zusammensetzung zur Verwendung nach Anspruch 10, wobei es sich bei dem Patienten um einen Menschen handelt.

14. Verwendung einer Zusammensetzung, die 5 mg bis 10 mg an Selegilin als Monoaminoxidase B inhibierende Komponente und 60 mg bis 250 mg an Disulfiram als Aldehyddehydrodrogenase I inhibierende Komponente umfasst, bei der Herstellung eines Arzneimittels zur Prävention, Behandlung oder Reduktion von Kokainabhängigkeit bei einem Patienten, der dessen bedarf.

## Revendications

1. Composition de commande d'effet stimulant comprenant une combinaison de sélégiline en tant que composant inhibant la monoamine oxydase (MOAB) et un composant inhibant l'aldéhyde déshydrogénase (ALDH), la composition étant formulée sous la forme de dose unitaire ; le stimulant comprenant de la cocaïne.

2. Composition selon la revendication 1, **caractérisée en ce que** l'inhibiteur de l'aldéhyde déshydrogénase est un inhibiteur de l'aldéhyde déshydrogénase I choisi dans le groupe constitué par le disulfiram, la coprine, le cyanamide, le 1-aminocyclopropanol, la daidzine, la céphalosporine, la sulfonylurée antidiabétique, le métronidazole et des métabolites ou analogues de ceux-ci qui présentent une activité inhibitrice de l'aldéhyde déshydrogénase I.

3. Composition selon la revendication 1 ou 2, dans laquelle l'aldéhyde déshydrogénase est le disulfirame ou un métabolite ou analogue de celui-ci qui présente une activité inhibitrice de l'aldéhyde déshydrogénase I.

4. Composition selon la revendication 1 ou 3, la composition comprenant une quantité de disulfirame choisie dans le groupe constitué par : environ 500 mg, environ 250 mg, environ 125 mg, environ 60 mg et environ 30 mg de disulfirame.

5. Composition selon la revendication 1, la composition comprenant une quantité de sélégiline choisie dans le groupe consistant par : environ 15 mg ou moins, environ 10 mg ou moins, environ 5 mg ou moins, environ 2,5 mg ou moins et environ 1 mg ou moins de sélégiline.

6. Composition selon la revendication 1, dans laquelle l'inhibiteur de la monoamine oxydase B est la sélégiline et l'inhibiteur de l'aldéhyde déshydrogénase I est le disulfirame.

7. Composition selon la revendication 1, comprenant une quantité quotidienne thérapeutiquement efficace de 5 mg à 10 mg de sélégiline et de 60 mg à 250 mg de disulfirame, respectivement.

8. Composition selon la revendication 7, dans laquelle la quantité quotidienne thérapeutiquement efficace de sélégiline est 5 mg à 10 mg et la quantité quotidienne thérapeutiquement efficace de disulfirame est de 250 mg.

9. Composition pharmaceutique comprenant une quantité thérapeutiquement efficace d'une combinaison de 5 mg à 10 mg de sélégiline en tant que composant inhibant la monoamine oxydase (MOAB) et de 60 mg à 250 mg de disulfirame en tant que composant inhibant l'aldéhyde déshydrogénase I (ALDH), la composition étant formulé sous la forme de dose unitaire ; et un excipient ou support pharmaceutiquement acceptable.

10. Composition comprenant de 5 mg à 10 mg de sélégiline, un composant inhibiteur de la monoamine oxydase B, et de 60 mg à 250 mg de disulfirame en tant que composant inhibiteur de l'aldéhyde déshydrogénase I, laquelle composition est destinée à être utilisée dans un procédé de prévention, de traitement ou de réduction de la dépendance à la cocaïne chez un patient en ayant besoin.

11. Composition destinée à être utilisée selon la revendication 10, la composition étant administrée par voie orale, parentérale ou transdermique.

12. Composition destinée à être utilisée selon la revendication 10, la composition étant administrée sous la forme d'une gélule, d'un comprimé ou d'un timbre transdermique.

13. Composition destinée à être utilisée selon la revendication 10, le patient étant un être humain.

14. Utilisation d'une composition comprenant de 5 mg à 10 mg de sélégiline, un composant inhibiteur de la monoamine oxydase B, et de 60 mg à 250 mg de disulfirame en tant que composant inhibiteur de l'aldéhyde déshydrogénase I, dans la fabrication d'un médicament destiné à prévenir, traiter ou réduire la dépendance à la cocaïne chez un patient en ayant besoin.
